# EUROPEAN PATENT SPECIFICATION

(11) **EP 2 758 319 B1**
(45) Date of publication and mention of the grant of the patent: **08.06.2016**
(21) Application number: 12759479.4
(22) Date of filing: 20.09.2012
(51) Int. Cl.: B65D 51/00, A61J 1/14

(54) **PACKAGING OF CONTRAST MEDIA**
VERPACKUNG VON KONTRASTMITTELN
EMBALLAGE DE PRODUITS DE CONTRASTE

(30) Priority: 21.09.2011 EP 11182144
(43) Date of publication of application: 30.07.2014
(73) Proprietor: GE Healthcare AS, 0401 Oslo (NO)
(72) Inventor: JACHWITZ, Bente, N-0401 Oslo (NO); GLØGÅRD, Christian, N-0401 Oslo (NO)
(74) Representative: Bannan, Sally
(86) International application number: PCT/EP2012/068475
(87) International publication number: WO 2013/041593

(56) References cited:
- EP-A1- 0 105 752
- WO-A1-00/73163
- WO-A2-2009/008734
- US-A- 4 321 306

## Description

The present invention relates to packaging material for contrast media and more specifically to packages comprising containers filled with contrast media and closed with a closure means. In a particular, the invention relates to packages material comprising closure means which provide an increased stability to the contrast media filled therein. The invention further relates to methods for improving the stability of such contrast media during storage, and in particular to methods including filling the contrast media in a container and closing the container with a closure means which provide an increased stability to the contrast media during storage.

All diagnostic imaging is based on the achievement of different signal levels from different structures within the body. Thus, in X-ray imaging for example, for a given body structure to be visible in the image, the X-ray attenuation by that structure must differ from that of the surrounding tissues. The difference in signal between the body structure and its surroundings is frequently termed contrast and much effort has been devoted to means of enhancing contrast in diagnostic imaging since the greater the contrast between a body structure and its surroundings the higher the quality of the images and the greater their value to the physician performing the diagnosis. The diagnostic quality of images is strongly dependent on the inherent noise level in the imaging procedure, and the ratio of the contrast level to the noise level can thus be seen to represent an effective diagnostic quality factor for diagnostic images. Achieving improvement in such a diagnostic quality factor has long been and still remains an important goal.

In techniques such as X-ray, one approach to improve the diagnostic quality factor has been to introduce contrast enhancing materials formulated as contrast media into the body region being imaged. Thus for X-ray, early examples of contrast agents were insoluble inorganic barium salts which enhanced X-ray attenuation in the body zones into which they distributed. For the last 50 years the field of X-ray contrast agents has been dominated by soluble iodine containing compounds. The clinical safety of iodinated X-ray contrast media has continuously been improved over the recent decades through development of new agents; from ionic monomers (Isopaque^{™}) to non-ionic monomers (e.g. Omnipaque^{™}) and non-ionic dimers (e.g. Visipaque^{™}). The utility of the contrast media is governed largely by its toxicity, by its diagnostic efficacy, by adverse effects it may have on the subject to which the contrast medium is administered, but also by the ease of production, storage and administration. The toxicity and adverse biological effects of a contrast medium are contributed to by the components of the formulation medium, i.e. of the diagnostic composition, e.g. the solvent or carrier as well as the contrast agent itself and its components such as ions for the ionic contrast agents and also by its metabolites.

The manufacture of non-ionic X-ray contrast media involves the production of the chemical drug, the active pharmaceutical ingredient (API), i.e. the contrast agent, followed by the formulation into the drug product, herein denoted the X-ray contrast media. In the preparation of an X-ray contrast media, the contrast agent is admixed with additives, such as salts, optionally after dispersion in a physiologically tolerable carrier. The contrast agent, such as a non-ionic iodinated compound, e.g. a non-ionic monomer or non-ionic dimer, has to be completely solved in the carrier when additives are included and the composition is prepared. A well-known process for preparing X- ray contrast medias includes heating the contrast agent in the carrier, such as water for injection, to ensure complete dissolution. For instance for the contrast media Visipaque the process includes dissolution of the contrast agent iodixanol in water for injection and heating to about 98 °C. Heating at this temperature for an adequate period of time ensures that the contrast agent is completely dissolved. Iodinated x-ray contrast media have in common that they are highly concentrated solutions and achieving sufficient solubility is a challenge. Further, different X-ray contrast agents have different solubility resulting in different challenges in the secondary production. For instance WO 2009/008734 of the applicant discloses a new class of compounds and their use as X-ray contrast agents. The compounds are dimers containing two linked iodinated phenyl groups. The bridge linking the two iodinated phenyl groups is a straight C3 to C8 alkylene chain optionally substituted by one to six -OH or -OCH₃ groups. Compound I, loforminol, which is one specific dimeric X-ray contrast agent, falling within the formula I of WO2009/008734, has been found by the applicant to have favourable properties.

Compound I: 5-[formyl-[3-[formyl-[3,5-bis(2,3-dihydroxypropylcarbamoyl)-2,4,6-triiodophenyl]amino]-2-hydroxypropyl]amino]-N,N'-bis(2,3-dihydroxypropyl)-2,4,6-triiodobenzene-1,3-dicarboxamide.

The injection solution of loforminol is highly supersaturated. A solution in which the concentration of the solute (Active Pharmaceutical Ingredient, API) exceeds the equilibrium solute concentration at a given temperature is said to be supersaturated.

This is possible because the solute does not precipitate immediately when the solution is cooled below the saturation temperature. Such solutions are denoted as supersaturated. Saturation temperature is the temperature where all solid API (amorphous and crystalline) apparently dissolves completely. As the solubility of loforminol decreases with decreasing temperature, the supersaturation increases. A nucleation, and hence precipitation, in the injection solution at storage conditions is strongly undesirable. In order to obtain a commercially acceptable product, with an acceptable shelf life, the drug product needs to stay clear of crystallization for a minimum of two years at 30°C. Ways for improving the physical stability of the solution, i.e. preventing the nucleation for a certain time at storage conditions, have been sought.

Supersaturated solutions are thermodynamically unstable, and are prone to nucleate and therefore precipitate on storage. So far the onset of the precipitation has been believed to depend mainly on the degree of supersaturation, presence of the crystals of the solute and foreign particles such as dust or other impurities, i.e. purity, and storage temperature of the solution. The onset of precipitation may e.g. be delayed by a proper treatment of the solution, such as by the secondary production process described in WO2011/117236 of the applicant, using temperature and pH regulation.

The applicant has now found that one factor that can impact crystallisation and hence the physical stability of a supersaturated solution is the surface properties of the material that the solution is exposed to, such as the packaging material.

US7,344,766 of Novo Nordisk A/S is directed to packaging of pharmaceuticals and more specifically presents a stopper made from a thermoplastice elastomer for the use in medical containers. The patent presents a combination of two stopper components that result in a reduced leakage of substances from a solution in a container closed with the stopper. The two components are a butyl based rubber and a polymer, such as a thermoplastic polymer, e.g. in an amount of 70-90 % by weight of the butyl based rubber and 10-30 % by weight of the thermoplastic polymer. The patent further suggests that conventional fillers, such as carbon black, clay, talc and white carbon may be added.

The present invention provides a solution to the stability problem of contrast media comprising a supersaturated solution, such that precipitation is reduced or avoided.

In a first aspect the invention provides an X-ray contrast agent in a container, in accordance with appended claim 1.

In a preferred embodiment the supersaturated solution comprises the contrast agent loforminol.

The solution of loforminol is a supersaturated solution, and the solution is a contrast media. The terms supersaturated solution, X-ray diagnostic composition and x-ray contrast media will be used interchangeably in this document and have the same meaning. With the container being closed with a closure means comprising Carbon Black it has been found that the physical stability of the contrast media, such as the solution of loforminol, is considerably increased.

Carbon Black [C.A.S No. 1333-86-4] is virtually pure elemental carbon in the form of colloidal particles. Its physical appearance is black, finely divided pellets or powder. Carbon Black has been used as a reinforcing agent in tires, but the use has also expanded to include pigmentation, ultraviolet stabilisation and as conductive agents in a variety of products. Carbon Blacks provide pigmentation, conductivity and UV protection for a number of coating applications. Carbon Blacks enhance formulations and deliver broad flexibility in meeting specific colour requirements. In rubber, Carbon Black is added both as a filler and as a strengthening or reinforcing agent. Carbon black can be broadly defined as very fine particulate aggregates of carbon possessing an amorphous quasi-graphitic molecular complex structure. The properties have a large effect on practical properties such as blackness and dispersibility. Carbon Black are classified and assigned a grade number based on surface area and structure measurements.

The surface activity of Carbon Black is influenced by the graphitic plane orientation as well as the number and type of organic side groups. On a molecular level, Carbon Black is composed of amorphous graphite layer planes created from the condensation of aromatic rings. The surface of Carbon Black contains numerous types of organic functional groups such as phenols, hydroxyls, lactones, carboxylic acids and quinones which contribute to the level of surface activity. The influence of Carbon Black on rubber can best be described in terms of processing and vulcanization properties. In choosing Carbon Black(s) for an elastomer formulation, the grade of Carbon Black and the degree of loading must be taken into consideration. The general effects of Carbon Black on any given rubber property can be summarized according to surface area (particle size), structure and loading level. It has now surprisingly been found that the complex surface structure and the various functional groups present on the surface of the Carbon Black particulate aggregates may prevent molecules from finding the orientation required for crystal growth and hence increase the physical stability of supersaturated solutions.

The applicant has now surprisingly found that inclusion of Carbon Black in the material of the closure means which are in contact with supersaturated solutions, affects the stability of the solutions positively, compared to stoppers not containing Carbon Black. Hence, it has been found that the physical stability of a supersaturated solution, such as a solution of loforminol, is substantially increased when this is filled in a container and closed with closure means comprising Carbon Black. The closure means of the invention have the effect that particle formation and growth is avoided in the supersaturated solution and the solution maintains stable, with no precipitation, in a considerable longer time than solutions filled in containers closed with closure means not comprising Carbon Blacks.

In one embodiment the container of the package is selected from the group of bottles, vials and syringes. The container may be of glass or plastic, such as of opaque or clear plastic, including plastic comprising polypropylene, cyclo-olefin polymers (COP) and cyclic olefin copolymers (COC). The container has a mouth. In a preferred embodiment the container is made of plastic, rather than glass.

The closure means of the package is selected from the group of stoppers, plunger stoppers, plugs, seals, caps, tops and corks, and combinations thereof. A part of the closure means is made of an elastomer and at least part of this is in physical contact with the content of the container, i.e. with the contrast media filled in the container. Preferably the closure means is a stopper or a plunger stopper, and such stopper is made of an elastomer.

In the embodiment wherein the closure means is a stopper, the stopper is removably inserted into the mouth of the container. Different stopper types and shapes exist. Two main shapes are the so called injection stoppers and the freeze-drier stoppers. Any stopper shapes, convenient for pharmaceutical packaging, fitting to vials (diameter of mouth is ≤ 20 mm) or bottles (diameter of mouth is > 20 mm) are covered by this invention. The stopper should provide good sealing properties and self-sealing properties, chemical stability, good properties of sterilizing under high temperature, and strong water-proof and damp-proof performance. The package may comprise further parts, such as further parts to close the container. For example, a cap may be overlaying the stopper, i.e. the stopper has an overseal. In one embodiment, said cap may comprise a removable portion which can be removed to gain access to said stopper. Such removable portion may have an engageable member for operation by a user to remove the removable portion. Such engageable member can e.g. comprise a ring upwardly spaced from the removable portion. Said cap may further comprise a wall extending generally about the periphery of the engageable member to protect it from accidental operation or entanglement. Said wall preferably has at least one opening there through. The cap further preferably comprises at least one projection, which acts as a pivot for said engageable member. The cap is preferably provided with a member which engages with said stopper when said package is closed to protect a defined region of said stopper from contamination.

In another embodiment, the package is a syringe, or also called a cartridge, and in this embodiment the container is the barrel of the syringe. A plunger, or piston, is movable within the barrel of the syringe to expel the contrast media through a tip thereof. The closure means comprising Carbon Black is a plunger stopper. Hence, the plunger of the syringe has a plunger stopper, i.e. an elastomer at the plunger end, which is in contact with the contrast media contained in the barrel, and this plunger stopper comprises Carbon Black. Preferably, the syringe is a single use syringe or a pre-filled syringe. The plunger provides both gliding force and sealability.

The contrast media filled in the container of the invention may be in a ready to use concentration or may be a concentrate form for dilution prior to administration. It may be desirable to make up the solution's tonicity by the addition of plasma cations so as to reduce the toxicity contribution that derives from the imbalance effects following bolus injection. In particular, addition of sodium, calcium, potassium and magnesium ions to provide a contrast medium isotonic with blood for all iodine concentrations is desirable and obtainable. The plasma cations may be provided in the form of salts with physiologically tolerable counterions, e.g. chloride, sulphate, phosphate, hydrogen carbonate etc., with plasma anions preferably being used.

Commercially available closure means, such as stoppers, are available in different materials. The closure means used in the invention should comply with the major pharmacopoeias, such as the US and EU pharmacopoeias. The closure means may be prepared from standard formulations or may be custom made. Commercially available "off the shelf" stoppers that are black and comprising Carbon Black have not been identified, but have been made available from the main suppliers of rubber stoppers on request. As the production of Carbon Black-containing closure means involves generation of finely divided black dust, such closure means would generally not be the first choice of the supplier. Off the shelf stoppers, such as grey or red rubber stoppers, would usually be sufficient and would be offered by the suppliers. Stoppers useful according to the invention may be available from the main suppliers, such as from West Pharmaceutical Systems, Helvoet Pharma, Stelmi or Daikyo Seiko, on request.

To the surprise of the inventors, testing a range of closure means of different materials, including stoppers for vials and bottles and plunger stoppers for syringes, the Carbon Black-containing closure means provided significantly increased physical stability to the contrast media. Well known components used in the materials of elastomeric stoppers are chlorobutyl and bromobutyl. The closure means of the package of the invention preferably comprises such halobutyl based rubber as the main component. As an alternative, or in addition, the closure means material may include other elastomers such as thermoplastic polymers such as those selected from polyisoprene, styrene butadiene rubber, polypropylene and polyethylene.

Hence, the Carbon Black is an additive to the main components of the material of the closure means. The colour of the material of the closure means can be used as an indicator for how much Carbon Black the material of the closure means comprise. The more Carbon Black, the darker the colour of the material of the closure means. A typical grey coloured stopper comprises about 0.13 % Carbon Black by weight. An addition of a mixture of titan oxide and Carbon Black to an elastomer may give a grey colour, while titan oxide only gives a white colour. It has been found that rubber stoppers with a black colour provide higher degree of stability to the contrast media in the container closed with the closure means, than stoppers of a lighter colour. In a preferred embodiment, the closure means of the invention is black. Hence, enough Carbon Black is present to colour it black and to provide the properties which other colour additives, such as titandioxide, do not provide. It is assumed that the material of the closure means used in the invention should comprise 0.1-5.0 % Carbon Blacks by weight, particularly for closure means which comprises a halobutyl based rubber. More preferably the material comprises 0.3-2.5 % Carbon Blacks by weight. In one embodiment, the material comprises about 1 % Carbon Blacks by weight. For other rubber based materials than the butyl based rubber material, a higher content of Carbon Black can be used, for instance closure means of the invention comprising a styrene based rubber may have a content of Carbon Black up to 21%. Such other rubber bases may be used in closure means because of improved compatibility between the container material and the closure means materials. The Food and Drug Administration (FDA, USA) sets an upper limit for the use of Carbon Blacks, and the levels should not exceed 2.5 % by weight of the polymer, provided that the preparation is by the impingement process. Other ingredients may be present in the formulation of the closure means material in addition to the elastomer and the Carbon Blacks, such as fillers, plastizers, antioxidants and colours.

In a preferred embodiment the closure means comprises chlorobutyl or bromobutyl rubber, and most preferably chlorobutyl. In one embodiment, the package of the invention is closed with a closure means of a material selected from the group of PH701/45 Carbon Black art. no.1071 and PH701/45 Carbon Black art. No. 4412. Some stoppers comprise a coating, such as a laminate, e.g. FluroTec®, and such stoppers may be used in the package of the invention, provided that they include Carbon Black in the elastomer.

In another embodiment, the supersaturated solution used in the invention is a low-osmolar contrast media (LOCM). Preferably the contrast agent of the contrast media is a non-ionic iodinated monomeric compound or a non-ionic iodinated dimeric compound, i.e. a compound comprising single triiodinated phenyl groups or a compound comprising two linked triiodinated phenyl groups. However, trimeric, tetrameric and pentameric compounds are also included. Relevant monomeric and dimeric compounds are provided by the applicant's application WO2010/079201. Particularly relevant monomeric compounds are described in WO97/00240 and in particular the compound BP257 of example 2, and additionally the commercially available compounds iopamidol, iomeprol, ioversol, iopromide, ioversol, iobitridol, iopentol and iohexol. Most particularly preferred are the compounds iopamidol and iohexol.

Particularly relevant dimeric compounds are compounds of formula (I) of two linked triiodinated phenyl groups, denoted non-ionic dimeric compounds,

R-N(CHO) -X-N(R⁶)-R Formula (I)

and salts or optical active isomers thereof,
wherein
X denotes a C₃ to C₈ straight or branched alkylene moiety optionally with one or two CH₂ moieties replaced by oxygen atoms, sulphur atoms or NR⁴ groups and wherein the alkylene moiety optionally is substituted by up to six -OR⁴ groups;
R⁴ denotes a hydrogen atom or a C₁ to C₄ straight or branched alkyl group;
R⁶ denotes a hydrogen atom or an acyl function, such as a formyl group; and
each R independently is the same or different and denotes a triiodinated phenyl group, preferably a 2,4,6-triiodinated phenyl group, further substituted by two groups R⁵ wherein each R⁵ is the same or different and denotes a hydrogen atom or a non-ionic hydrophilic moiety, provided that at least one R⁵ group in the compound of formula (II) is a hydrophilic moiety. Preferred groups and compounds are outlined in applications WO2010/079201 and WO2009/008734.

In the embodiment wherein the supersaturated solution comprises another contrast agent than loforminol, the container used is preferably a syringe, wherein the plunger stopper comprises Carbon Black. Most preferably, the syringe is a prefilled syringe filled with a supersaturated solution of a contrast media.

The surface size of the closure means which the contrast media is exposed to, that is, is in physical contact with may impact the precipitation, but no direct relation has been found. Other parameters like the shape of the closure means and the composition of the closure material seem more important.

The X-ray contrast media can be administered by injection or infusion, e.g. by intravascular administration. If the container of the package is a bottle and the closure means is a stopper, the stopper can be pierced by a hypodermic needle, a infusion spike or similar to withdraw the content. Alternatively, the entire stopper can be removed, to enable pouring or the insertion of a quill or straw to load an autoinjector. In one embodiment, the X-ray diagnostic composition is administered as a rapid intravascular injection, in another embodiment it is administered as a steady infusion.

In a second aspect the invention provides a method of improving the stability of a supersaturated solution of an X-ray contrast agent, in accordance with appended claim 11.

As for the first aspect, the X-ray contrast agent is a non-ionic iodinated monomeric compound or a non-ionic iodinated dimeric compound, and is most preferably ioforminol. And as for the first aspect, the container of the package is selected from the group of bottles, vials and syringes. The bottles may be of glass or plastic, such as of opaque or clear plastic. The closure means of the package is selected from the group of a stoppers, plunger stoppers, plugs, seals, caps, tops and corks. Preferably the closure means is a stopper or a plunger stopper.

In one embodiment, the method of the invention further includes a sterilisation step. Preferably such sterilization includes heat treatment after filling. It is very difficult to achieve a particle free atmosphere during the filling of the containers. The containers may also contain tiny particles, in spite of washing of the containers. A final heat treatment, e.g. steam sterilization, of the filled and sealed containers at a suitable temperature, above the saturation temperature of the contrast agent, is critical with respect of dissolving foreign particles brought to the containers by dust and to deactivate the insoluble foreign particles present in the solution.

This aspect includes the same features and fall-backs as the first aspect of the invention.

It has been seen that with the method of the invention, closing the container with a closure means comprising Carbon Black, the physical stability of the contrast media is increased, and the contrast media may be stored for a longer time. Various closure means have been tested. Hence, bottles and syringes comprising a supersaturated solution of ioforminol have been closed with various stoppers and plunger stoppers respectively, at different temperatures, and stored. With the package and the method of the invention the results clearly show that solutions in containers closed with black Carbon Black-containing stoppers provided a longer physical stability. The filled containers were stored at 25, 40 and 50 °C. For the solutions closed with non-Carbon Black containing stoppers precipitation started much earlier than for the Carbon Black-containing stoppers. For FluroTec® coated stoppers the precipitation started already after 2-3 weeks, even at the lower temperatures. For the Carbon Black stoppers there were, at the 25 and 40 °C degrees storage, no bottles closed with a Carbon Black type stopper that had any signs of precipitation even after 24 weeks (5 months). With the package and method of the invention it is believed the supersaturated solution can be stored at 3 years, or at least 2.5 years, or at least 2 years without any signs of precipitation.

The invention is illustrated with reference to the following non-limiting examples.

### Examples:

### Example 1: Stability testing of loforminol solution at different temperatures and with different closure means.

An experimental study was designed to find out how primary packaging material impacted on the physical stability of loforminol 320 mgl/ml.

Different vials and bottles (glass, polypropylene, clear plastic) filled with an loforminol 320 mgl/ml formulation with different stoppers were included in the study. The samples (vials, PLUSPAK™ and Clear Plastic bottles) with different stoppers (closures) were stored at 25, 40 and 50 °C. The stoppers were made of bromobutyl-and chlorobutyl rubber, some being black, comprising Carbon Black (black stopper), and some comprising other red-brown and grey material, and some were FluroTec® coated. For each study 20 or 30 identical packages were tested. The containers were reviewed weekly for 6 months. From 6 months, the container were reviewed monthly and from 12 months the containers are reviewed every third month.

An extract of the results is provided in table 1 below. Results after 15 months storage at 40 °C and 50 °C clearly show differences in crystallisation between the samples depending on the materials used in the stoppers. The loforminol 320 mgl/ml solution in all vials using Carbon Black stoppers (black) were found to be more stable (no or few containers with crystals observed) than vials using for instance FluroTec® coated stoppers.

The results for the glass and clear plastic bottles show that the material of the stoppers clearly impacts the physical stability of loforminol 320 mgl/ml. At 50 °C the results range from precipitation start after 2 weeks with approx. 80 % of samples precipitated where FluroTec® coated stoppers were used, to no or only 1-3 containers with precipitation for samples where Carbon Black stoppers were used.
PP: Polypropylene
COP: Cyclo-olefin polymer
COC: Cyclic olefin copolymer

**Table 1:**

| | | Closure | | | | |
|---|---|---|---|---|---|---|
| No | Container | Butyl rubber type | Lamination | Colour | First sign of precipitation, at °C | Result after 15 months (no. of units with precipitation of x number of samples) |
| | | | | | 25°C: --- | 0 of 20 |
| 1 | Glass | Chloro | No | Grey | 40°C: 7 months | 8 of 20 |
| | | | | | 50°C: 9 weeks | > 20 of 30 |
| | | | | | 25°C: --- | 0 of 20 |
| 2 | Glass | Chloro | No | Grey | 40°C: 10 months | 7 of 20 |
| | | | | | 50°C:12 weeks | > 20 of 30 |
| | | | | | 25°C: --- | 0 of 20 |
| 3 | Glass | Bromo | Yes | Grey | 40°C: 22 weeks | >14 of 20 |
| | | | | | 50°C: 6 weeks | > 20 of 30 |
| | | | | | 25°C: --- | 0 of 20 |
| 4 | Glass | Chloro | No | Redbr | 40°C: --- | 0 of 20 |
| | | | | | 50°C: 10 weeks | >13 of 30 |
| | | | | | 25°C: --- | 0 of 20 |
| 5 | Glass | Chloro | No | Grey | 40°C: 8 months | 7 of 20 |
| | | | | | 50°C: 11 weeks | >14 of 30 |
| | | | | | 25°C: --- | 0 of 20 |
| 6 | Glass | Chloro | No | Black | 40°C: 15 months | 5 of 20 |
| | | | | | 50°C:15 weeks | >15 of 30 |
| | | | | | 25°C: --- | 0 of 20 |
| 7 | Glass | Chloro | Yes | Grey | 40°C: 12 weeks | >15 of 20 |
| | | | | | 50°C: 2 weeks | 30 of 30 |
| | | | | | 25°C: --- | 0 of 20 |
| 8 | Glass | Bromo | No | Grey | 40°C: 24 weeks | >15 of 20 |
| | | | | | 50°C: 5 weeks | 30 of 30 |
| | | | | | 25°C: --- | 0 of 20 |
| 9 | PP | Chloro | No | Grey | 40°C: 8 months | 9 of 20 |
| | | | | | 50°C: 9 weeks | > 20 of 30 |
| | | | | | 25°C: --- | 0 of 20 |
| 10 | PP | Chloro | No | Grey | 40°C: 22 weeks | >15 of 20 |
| | | | | | 50°C: 8 weeks | 30 of 30 |
| | | | | | 25°C: --- | 0 of 20 |
| 11 | PP | Chloro | No | Black | 40°C: 15 months | 1 of 20 |
| | | | | | 50°C: 15 weeks | 1 of 30¹ |
| | | | | | 25°C: --- | 0 of 20 |
| 12 | COP | Chloro | No | Grey | 40°C: 8 months | >11 of 20 |
| | | | | | 50°C: 7 weeks | > 25 of 30 |
| 13 | COP | Chloro | No | Grey | 25°C: --- | 0 of 20 |
| | | | | | 40°C: 27 weeks | >12 of 20 |
| | | | | | 50°C: 7 weeks | > 20 of 30 |
| | | | | | 25°C: --- | 0 of 20 |
| 14 | COP | Chloro | No | Black | 40°C: 11 months | 2 of 20¹ |
| | | | | | 50°C: 15 months | 1 of 20 |
| | | | | | 25°C: --- | 0 of 20 |
| 15 | COC | Chloro | No | Grey | 40°C: 27 weeks | 17 of 20 |
| | | | | | 50°C: 10 weeks | > 20 of 30 |
| | | | | | 25°C: --- | 0 of 20 |
| 16 | COC | Chloro | No | Grey | 40°C: 22 weeks | >15 of 20 |
| | | | | | 50°C:6 weeks | > 25 of 30 |
| | | | | | 25°C: --- | 0 of 20 |
| 17 | COC | Chloro | No | Black | 40°C: 11 months | 2 of 20 |
| | | | | | 50°C: 10 weeks | 3 of 20² |

| | | | | | | |
|---|---|---|---|---|---|---|
| 1 this sample is considered random precipitation and no trend of increasing number of units with precipitation 2 new units with precipitation were first observed after 19 weeks | | | | | | |

## Claims

1. A stabilised supersaturated solution of an X-ray contrast agent in a container closed with a closure means, **characterised in that** said closure means comprises Carbon Black, wherein said closure means comprising Carbon Black provides increased physical stability to the solution.

2. A stabilised supersaturated solution of an X-ray contrast agent as claimed in claim 1 wherein the supersaturated solution comprises the contrast agent loforminol.

3. A stabilised supersaturated solution of an X-ray contrast agent as claimed in claim 1 or 2 wherein the container is selected from the group of bottles, vials and syringes.

4. A stabilised supersaturated solution of an X-ray contrast agent as claimed in any of claims 1 to 3 wherein the closure means is selected from the group of stoppers, plunger stoppers, plugs, seals, caps, tops and corks.

5. A stabilised supersaturated solution of an X-ray contrast agent as claimed in any of claims 1 to 4 wherein the closure means is coloured black by the Carbon Black.

6. A stabilised supersaturated solution of an X-ray contrast agent as claimed in any of claims 1 to 5 wherein the material of the closure means comprises up to 21 % Carbon blacks.

7. A stabilised supersaturated solution of an X-ray contrast agent as claimed in any of claims 1 to 6 wherein the material of the closure means comprises 0.1-5.0 % Carbon Blacks.

8. A stabilised supersaturated solution of an X-ray contrast agent as claimed in any of claims 1 to 7 wherein the closure means comprises a halobutyl based rubber.

9. A stabilised supersaturated solution of an X-ray contrast agent as claimed in claim 6 wherein the closure means comprises a styrene based rubber.

10. A stabilised supersaturated solution of an X-ray contrast agent as claimed in any of the claims 1 to 9 wherein the closure means is a stopper and the package further comprises a cap overlying the stopper.

11. A method of stabilising a supersaturated solution of an X-ray contrast agent, wherein the method includes filling the solution into a container and **characterised by** closing the container with a closure means comprising Carbon Black and wherein said closure means comprising Carbon Black provides increased physical stability to the solution.

12. A method as claimed in claim 11 using a package as claimed in any of claims 1 to 10.

13. A method as claimed in claims 11 or 12 further including a sterilisation step.

## Patentansprüche

1. Stabilisierte übersättigte Lösung eines Röntgen-Kontrastmittels in einem Behälter, der mit einem Verschlussmittel verschlossen ist, **dadurch gekennzeichnet, dass** das Verschlussmittel Kohlenschwarz umfasst, wobei das Verschlussmittel, das Kohlenschwarz umfasst, der Lösung eine erhöhte physikalische Stabilität zur Verfügung stellt.

2. Stabilisierte übersättigte Lösung eines Röntgen-Kontrastmittels nach Anspruch 1, wobei die übersättigte Lösung das Kontrastmittel Ioforminol umfasst.

3. Stabilisierte übersättigte Lösung eines Röntgen-Kontrastmittels nach Anspruch 1 oder 2, wobei der Behälter ausgewählt ist aus der Gruppe bestehend aus Flaschen, Phiolen und Spritzen.

4. Stabilisierte übersättigte Lösung eines Röntgen-Kontrastmittels nach einem der Ansprüche 1 bis 3, wobei das Verschlussmittel ausgewählt ist aus der Gruppe bestehend aus Stopfen, Kolbenstopfen, Stöpseln, Versiegelungen, Verschlüssen, Deckeln und Korken.

5. Stabilisierte übersättigte Lösung eines Röntgen-Kontrastmittels nach einem der Ansprüche 1 bis 4, wobei das Verschlussmittel durch das Kohlenstoffschwarz schwarz gefärbt ist.

6. Stabilisierte übersättigte Lösung eines Röntgen-Kontrastmittels nach einem der Ansprüche 1 bis 5, wobei das Material des Verschlussmittels bis zu 21 % Kohlenstoffschwarz umfasst.

7. Stabilisierte übersättigte Lösung eines Röntgen-Kontrastmittels nach einem der Ansprüche 1 bis 6, wobei das Material des Verschlussmittels 0,1 bis 5,0 % Kohlenstoffschwarz umfasst.

8. Stabilisierte übersättigte Lösung eines Röntgen-Kontrastmittels nach einem der Ansprüche 1 bis 7, wobei das Verschlussmittel einen auf Halobutyl basierenden Kautschuk umfasst.

9. Stabilisierte übersättigte Lösung eines Röntgen-Kontrastmittels nach Anspruch 6, wobei das Verschlussmittel einen auf Styrol basierenden Kautschuk umfasst.

10. Stabilisierte übersättigte Lösung eines Röntgen-Kontrastmittels nach einem der Ansprüche 1 bis 9, wobei das Verschlussmittel ein Stopfen ist und die Verpackung ferner einen Verschluss umfasst, der den Stopfen überdeckt.

11. Verfahren zur Stabilisierung einer übersättigten Lösung eines Röntgen-Kontrastmittels, wobei das Verfahren das Füllen der Lösung in einen Behälter beinhaltet, und durch das Verschließen des Behälters mit einem Verschlussmittel, das Kohlenschwarz umfasst, gekennzeichnet ist, und wobei das Verschlussmittel, das Kohlenschwarz umfasst, der Lösung einer erhöhte physikalische Stabilität zur Verfügung stellt.

12. Verfahren nach Anspruch 11, das eine Verpackung nach einem der Ansprüche 1 bis 10 verwendet.

13. Verfahren nach einem der Ansprüche 11 oder 12, ferner beinhaltend einen Schritt der Sterilisation.

## Revendications

1. Solution sursaturée stabilisée d'un agent de contraste aux rayons X dans un contenant fermé par un moyen de fermeture, **caractérisée en ce que** le moyen de fermeture comprend du noir de carbone, dans lequel ledit moyen de fermeture comprenant du noir de carbone assure une stabilité physique renforcée à la solution.

2. Solution sursaturée stabilisée d'un agent de contraste aux rayons X selon la revendication 1, dans laquelle la solution sursaturée comprend l'agent de contraste Ioforminol.

3. Solution sursaturée stabilisée d'un agent de contraste aux rayons X selon la revendication 1 ou la revendication 2, dans laquelle le contenant est choisi dans le groupe des bouteilles, des flacons et des seringues.

4. Solution sursaturée stabilisée d'un agent de contraste aux rayons X selon l'une quelconque des revendications 1 à 3, dans laquelle le moyen de fermeture est choisi dans le groupe des obturateurs, des obturateurs de plongeurs, des tampons, des joints étanches, des capuchons, des couvercles et des bouchons de liège.

5. Solution sursaturée stabilisée d'un agent de contraste aux rayons X selon l'une quelconque des revendications 1 à 4, dans laquelle le moyen de fermeture est coloré en noir par le noir de carbone.

6. Solution sursaturée stabilisée d'un agent de contraste aux rayons X selon l'une quelconque des revendications 1 à 5, dans laquelle le matériau du moyen de fermeture comprend jusqu'à 21 % de noir de carbone.

7. Solution sursaturée stabilisée d'un agent de contraste aux rayons X selon l'une quelconque des revendications 1 à 6, dans laquelle le matériau du moyen de fermeture comprend 0,1 à 5,0 % de noir de carbone.

8. Solution sursaturée stabilisée d'un agent de contraste aux rayons X selon l'une quelconque des revendications 1 à 7, dans laquelle le moyen de fermeture comprend du caoutchouc à base d'halobutyle.

9. Solution sursaturée stabilisée d'un agent de contraste aux rayons X selon la revendication 6, dans laquelle le moyen de fermeture comprend un caoutchouc à base de styrène.

10. Solution sursaturée stabilisée d'un agent de contraste aux rayons X selon l'une quelconque des revendications 1 à 9, dans laquelle le moyen de fermeture est un obturateur et l'emballage comprend en outre un capuchon recouvrant l'obturateur.

11. Procédé de stabilisation d'une solution sursaturée d'un agent de contraste aux rayons X, dans lequel le procédé comprend le remplissage d'un contenant par la solution et **caractérisé par** la fermeture du contenant par un moyen de fermeture comprenant du noir de carbone et dans lequel ledit moyen de fermeture comprenant du noir de carbone assure une stabilité physique renforcée à la solution.

12. Procédé selon la revendication 11 utilisant un emballage selon l'une quelconque des revendications 1 à 10.

13. Procédé selon les revendications 11 ou 12 comprenant en outre une étape de stérilisation.
